# EUROPEAN PATENT APPLICATION

(11) **EP 4 748 879 A1**
(43) Date of publication of application: **27.05.2026**
(21) Application number: 24842050.7
(22) Date of filing: 21.05.2024
(51) Int. Cl.: C08K 5/18, C08L 7/00, C08L 9/00

(54) **ANTIOXIDANT COMPOSITION HAVING GOOD OZONE AGING RESISTANCE, AND RUBBER COMPOSITION AND USE THEREOF**

(30) Priority: 19.07.2023 CN 202310890889
(71) Applicant: Sennics Co., Ltd., Shanghai, 200120 (CN)
(72) Inventor: GAO, Yang, Shanghai 200120 (CN); LI, Liang, Shanghai 201203 (CN); GUO, Xiangyun, Shanghai 200120 (CN)
(74) Representative: V.O.
(86) International application number: PCT/CN2024/094397
(87) International publication number: WO 2025/016050

(57) **Abstract**

Provided in the present invention are an antioxidant composition comprising two or more compounds of formula A, a rubber composition comprising the antioxidant composition, and the use of the rubber composition. The compounds of formula A are as described in the specification. The antioxidant composition of the present invention can significantly improve the static ozone aging resistance and dynamic ozone aging resistance of a tire rubber material, and the other properties thereof such as thermo-oxidative aging resistance can also be kept consistent with existing comprehensive antioxidants.

## Description

### TECHNICAL FIELD

The present invention belongs to the field of rubber materials and specifically relates to an antidegradant composition with excellent ozone aging resistance, a rubber composition, and use thereof.

### BACKGROUND

With the development of society and the progress of the automotive industry, the requirements for automobile tires are increasingly high. A tire is a rubber product. During processing, storage and use, rubber materials undergo structural changes and gradually lose their original excellent properties due to the influence of various factors such as light, oxygen, ozone, high temperature, and chemical corrosion-this is the aging phenomenon of rubber. In particular, rubbers containing double bonds and their products are prone to ozone aging when used or stored in an air environment containing ozone. Ozone aging can be manifested as: the formation of cracks and fissures perpendicular to the direction of stress on the surface of materials or products; the gradual decrease in mechanical properties of the materials such as tensile strength and elongation and the deterioration of service performance, etc. To this end, it is necessary to add antidegradants to tires to mitigate the impact of various types of aging on their performance. Among the existing tire antidegradants, the main structures are aniline-based, quinoline-based, and triazine-based.

Tires develop cracks when attacked by ozone in the air during static storage and vehicle operation. This situation may occur when tires are stored in warehouses, and such cracks will affect the appearance and performance of the tires, rendering them unmarketable and unusable. If these microcracks appear on tires on vehicles, they will expand into tire failure points during subsequent use, shortening the service life of the tires and even causing accidents while driving. Existing comprehensive antidegradants provide balanced protection for various properties, but their ozone protection performance is not outstanding.

### SUMMARY OF THE INVENTION

Aiming at the problems existing in the prior art, the present invention provides an antidegradant composition comprising two or more compounds of formula A, a rubber composition comprising the antidegradant composition, and uses thereof. The combined use of two or more compounds of formula A can significantly improve the static ozone aging resistance and dynamic ozone aging resistance of tire rubber materials, while other properties such as thermo-oxidative aging resistance can also maintain consistency with existing comprehensive antidegradants. This helps to effectively address the ozone aging phenomenon of tires, thereby prolonging the service life of tires and enhancing the safety guarantee during tire use.

Specifically, one aspect of the present invention provides an antidegradant composition, wherein the antidegradant composition comprises two or more compounds of formula A: 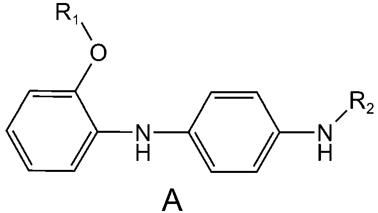 in formula A, R₁ and R₂ are each independently selected from C1-C8 alkyl.

In one or more embodiments, in formula A, R₁ is selected from C1-C2 alkyl, and R₂ is selected from C3-C6 alkyl.

In one or more embodiments, the antidegradant composition comprises two or more compounds selected from a compound of formula I, a compound of formula II, a compound of formula IV, a compound of formula V, a compound of formula VII, and a compound of formula VIII:

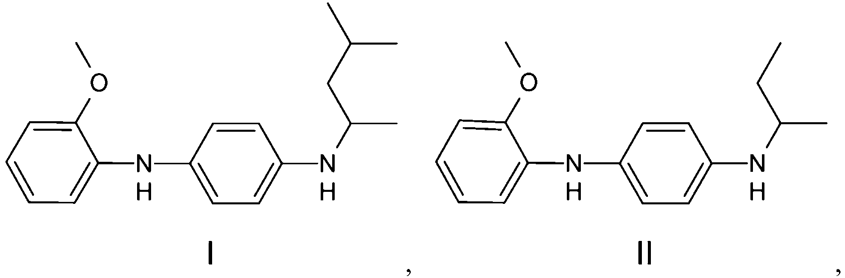

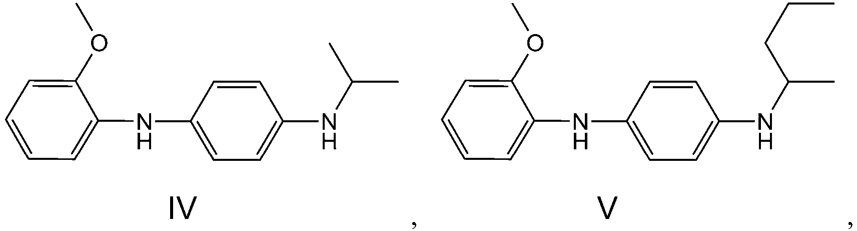

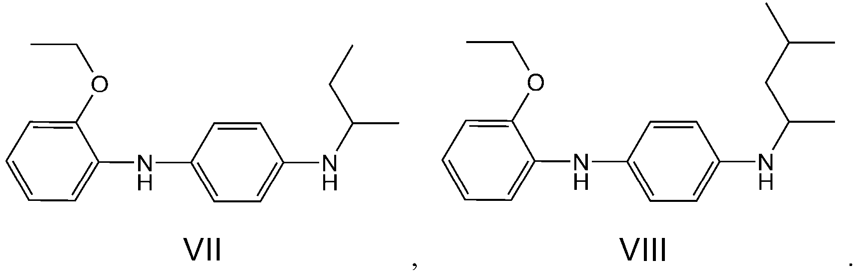

In one or more embodiments, the antidegradant composition comprises a compound of formula I and a compound of formula II, or comprises a compound of formula IV and a compound of formula V, or comprises a compound of formula VII and a compound of formula VIII.

In one or more embodiments, in the antidegradant composition, a mass ratio of a compound of formula I to a compound of formula II, a mass ratio of a compound of formula IV to a compound of formula V, or a mass ratio of a compound of formula VII to a compound of formula VIII is 1:4 to 4:1.

Another aspect of the present invention provides a rubber composition, wherein raw materials of the rubber composition comprise 100 parts by mass of a diene elastomer and 1 to 5 parts by mass of the antidegradant composition according to any embodiment herein.

In one or more embodiments, raw materials of the rubber composition comprise 2 to 3 parts by mass of the antidegradant composition.

In one or more embodiments, the diene elastomer comprises natural rubber and butadiene rubber; preferably, a mass ratio of the natural rubber to the butadiene rubber is 1:2 to 2:1.

In one or more embodiments, the raw materials of the rubber composition further comprise 30 to 70 parts by mass of a reinforcing filler; preferably, the reinforcing filler is carbon black.

In one or more embodiments, the raw materials of the rubber composition further comprise 1 to 10 parts by mass of an activator; preferably, the activator is zinc oxide.

In one or more embodiments, the raw materials of the rubber composition further comprise 2 to 15 parts by mass of a softener; preferably, the softener comprises aromatic oil and stearic acid in a mass ratio of 1:1 to 5:1.

In one or more embodiments, the raw materials of the rubber composition further comprise 0.5 to 3 parts by mass of sulfur.

In one or more embodiments, the raw materials of the rubber composition further comprise 0.2 to 2 parts by mass of an accelerator, and the accelerator is preferably N-tert-butylbenzothiazole-2-sulphenamide.

Another aspect of the present invention provides a rubber product, wherein the rubber product comprises a rubber composition according to any embodiment herein; preferably, the rubber product is a tire.

The present invention further provides a use of an antidegradant composition according to any embodiment herein in improving the ozone aging resistance of a rubber composition or a rubber product.

### DETAILED DESCRIPTION OF THE INVENTION

To enable those skilled in the art to understand the features and effects of the present invention, the following is a general description and definition of terms and words mentioned in the specification and claims. Unless otherwise specified, all technical and scientific terms used herein are intended to be the ordinary meaning of the knowledge of the present invention by those skilled in the art, and in case of a conflict, the definition of this specification shall prevail.

The theories or mechanisms described and disclosed herein, whether right or wrong, should not limit the scope of the present invention in any way, i.e., the content of the present invention may be practiced without limitation to any particular theory or mechanism.

Herein, the terms "comprising", "including", "containing" and the like encompass terms "consisting essentially of" and "consisting of". For example, where it is disclosed herein that "A comprises B and C", "A consists essentially of B and C" and "A consists of B and C" should be considered to be disclosed herein.

Herein, all features, such as numerical values, quantities, amounts and concentrations, which are defined by numerical ranges or percentage ranges, are only for the sake of simplicity and convenience. Accordingly, the recitation of numerical ranges or percentage ranges shall be construed as covering and specifically disclosing all possible sub-ranges and individual values (including integers and fractions) in the range.

Herein, unless otherwise specified, a percentage refers to a mass percentage, and a ratio refers to a mass ratio.

Herein, when embodiments or Examples are described, it should be understood that they are not intended to limit the disclosure to these embodiments or Examples. On the contrary, all alternatives, improvements and equivalents of the methods and materials described in the present disclosure can be covered within the scope defined by the claims.

Herein, for the sake of brevity of description, all possible combinations of various technical features in the various embodiments or Examples are not described. Therefore, as long as there is no contradiction in the combination of these technical features, the various technical features in the various embodiments or Examples can be combined in any combination, and all possible combinations should be considered to be within the scope of this specification.

The present invention has found that the combined use of two or more compounds of formula A-particularly the combined use of a compound of formula I and a compound of formula II, the combined use of a compound of formula IV and a compound of formula V, and the combined use of a compound of formula VII and a compound of formula VIII-in rubber compositions, especially in tire rubber compositions can significantly improve the ozone aging resistance of the rubber composition during static and dynamic processes, thereby prolonging the service life and enhancing the safety guarantee during use. Compared with the separate use of antidegradant 6PPD or only one compound of formula A, the combined use of two or more compounds of formula A-particularly the combined use of a compound of formula I and a compound of formula II, the combined use of a compound of formula IV and a compound of formula V, and the combined use of a compound of formula VII and a compound of formula VIII-exhibits an unexpected synergistic effect in improving the ozone aging resistance of the rubber composition.

In the present invention, the compound of formula A has the following structure: 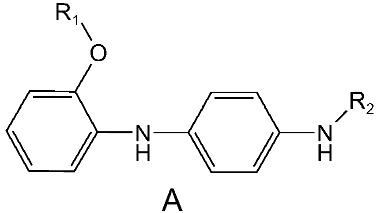 in formula A, R₁ and R₂ are each independently selected from C1-C8 alkyl.

Herein, "alkyl" refers to a linear or branched monovalent saturated hydrocarbon group. The alkyl group may contain 1 to 8 carbon atoms (C1-C8 alkyl). Examples of the alkyl group include, but are not limited to, methyl, ethyl, propyl, isopropyl, 1-methylpropyl, isobutyl, 1-methylbutyl, and 1,3-dimethylbutyl.

In some embodiments, in formula A, R₁ is selected from C1-C4 alkyl, for example, C1-C2 alkyl.

In some embodiments, in formula A, R₂ is selected from C3-C6 alkyl, such as isopropyl, 1-methylpropyl, 1-methylbutyl, and 1,3-dimethylbutyl, preferably selected from C4-C6 alkyl.

In some preferred embodiments, in formula A, R₁ is selected from C1-C2 alkyl; R₂ is selected from C3-C6 alkyl or C4-C6 alkyl, for example, selected from isopropyl, 1-methylpropyl, 1-methylbutyl, and 1,3-dimethylbutyl.

In the present invention, a compound of formula A may be prepared by a method comprising the following steps:
(1) performing a condensation reaction of a compound of formula B with nitrobenzene in the presence of a first catalyst to obtain a condensate comprising a compound of formula C and/or a compound of formula C', and then carrying out a reduction reaction of the condensate in the presence of H₂ and a second catalyst to obtain a compound of formula D; 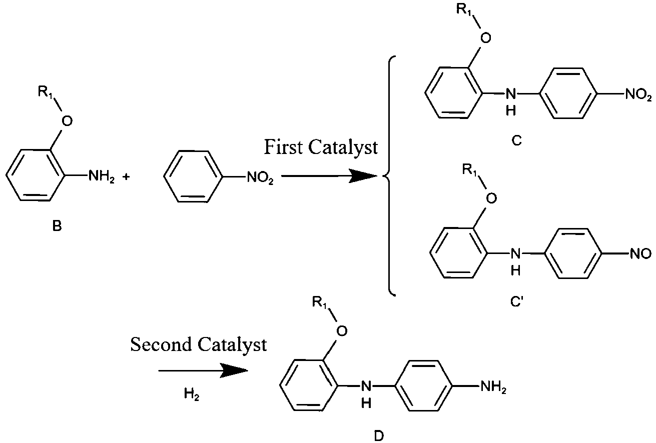
(2) conducting a reductive alkylation reaction of a compound of formula D with a compound of formula E in the presence of H₂ and a third catalyst to obtain a compound of formula A; 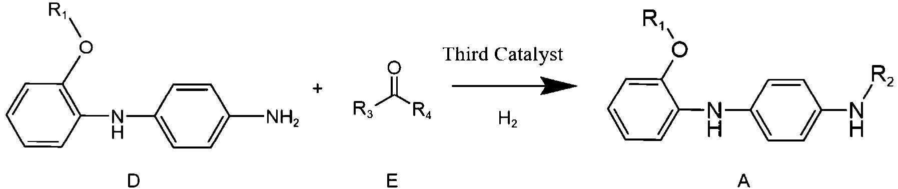
in formula A, formula B, formula C and formula D, R₁ and R₂ are as described in any embodiment herein; in formula E, R₃ and R₄ are each independently selected from H and C1-C7 alkyl. Those skilled in the art can determine the suitable R₄ and R₅ in formula F based on R₃ contained in a compound of formula A.

The first catalyst used in step (1) may be one or more selected from the group consisting of alkali metal hydroxides, alkali metal alkoxides, quaternary ammonium hydroxides, and combinations of alkali metal hydroxides with tetraalkylammonium halides. Alkali metal hydroxides suitable for the present invention include sodium hydroxide, potassium hydroxide, lithium hydroxide, and the like. Alkali metal alkoxides suitable for the present invention include sodium methoxide, potassium methoxide, sodium ethoxide, potassium ethoxide, sodium tert-butoxide, potassium tert-butoxide, sodium tert-pentoxide, potassium tert-pentoxide, and the like. Quaternary ammonium hydroxides are a class of compounds with a general formula of R¹₄NOH, wherein R¹ represents four identical or different aliphatic hydrocarbon groups or aromatic hydrocarbon groups. R₁ in the quaternary ammonium hydroxides suitable for the present invention may be one or more selected from methyl, ethyl, propyl, butyl, and the like. Examples of quaternary ammonium hydroxides suitable for the present invention include tetramethylammonium hydroxide, tetraethylammonium hydroxide, tetrabutylammonium hydroxide, and the like. The first catalyst may also be a combination of an alkali metal hydroxide and a tetraalkylammonium halide. Tetraalkylammonium halides have the general formula R²₄NX, where R² represents four identical or different aliphatic hydrocarbon groups or aromatic hydrocarbon groups, e.g., methyl, ethyl, propyl, butyl, etc., and X represents a halogen atom, e.g., fluorine, chlorine, bromine, iodine. Examples of combinations of alkali metal hydroxides and tetraalkylammonium halides include sodium hydroxide and tetrabutylammonium bromide, and the like.

A molar ratio of a first catalyst to a compound of formula B may be 0.1:1 to 2:1, preferably 0.9:1 to 1.1:1, for example, 1.05:1, 1.1:1, and 1.5:1. In some embodiments, in step (1), first, a compound of formula B is subjected to form a salt with a first catalyst, and then nitrobenzene is added dropwise to carry out a condensation reaction.

In step (1), a condensate obtained from a condensation reaction of a compound of formula B with nitrobenzene in the presence of a first catalyst may be one or both of a compound represented by formula C and a compound represented by formula C', and may further contain an azobenzene compound.

In step (1), a molar ratio of a compound of formula B to nitrobenzene may be 2:1 to 15:1, preferably 4:1 to 10:1, more preferably 5:1 to 8:1, for example, 6:1, and 7:1.

In step (1), a condensation reaction may be carried out at a temperature of 40°C to 90°C, preferably 65°C to 85°C; for example, a reaction temperature may be 60°C, 70°C, 75°C, or 80°C. The condensation reaction shall be carried out under vacuum conditions, with a pressure range of -0.09MPa to -0.1MPa.

The second catalyst used in step (1) may be a porous metal catalyst or a supported metal catalyst. Porous metal catalysts are also known as spongy metal catalysts. Porous metal catalysts suitable for the present invention include Raney nickel (also known as skeletal nickel), Raney cobalt, Raney copper, and the like. Supported metal catalysts comprise a metal as the catalytically active center and a support for loading the metal. The metal in the supported metal catalysts suitable for the present invention may be nickel, cobalt, copper, platinum, palladium, ruthenium, rhodium, or the like; the support may be carbon, alumina, silica gel, molecular sieve, or the like, and the carbon used as the support may be activated carbon. A molar ratio of the metal in a second catalyst to a condensate may be 0.0001:1 to 0.2:1.

In step (1), a condensate generated from a condensation reaction undergoes a hydrogenation reduction reaction in the presence of a second catalyst to produce a compound of formula D. In step (1), a reduction reaction may be carried out at a temperature of 40°C to 120°C, preferably 60°C to 90°C; for example, a reaction temperature may be 70°C, 75°C, and 80°C. A pressure of hydrogen in a reduction reaction may be 0.5MPa to 5MPa, for example, 1MPa, 1.5MPa, 2MPa, and 2.5MPa.

In step (1), a compound of formula B itself may be used as a solvent, or solvents such as toluene and xylene may also be used. After the completion of the reaction in step (1), the reaction solution is subjected to filtration, water washing, and phase separation; an organic phase is then subjected to vacuum distillation to remove light components, thereby obtaining a compound of formula D.

The third catalyst used in Step (2) may be the aforementioned supported metal catalyst, such as Pt/C. A molar ratio of the metal in a third catalyst to a compound of formula D may be 0.0001:1 to 0.2:1.

In step (2), the carbon atom of the carbonyl in a compound of formula E is linked to the nitrogen atom of the amino in a compound of formula D after the reaction. Therefore, a suitable compound of formula E can be selected for the reaction according to the R₃ group contained in a compound of formula A to be prepared. A molar ratio of a compound of formula E to a compound of formula D may be 1:1 to 15:1, for example, 2:1, 3:1, 5:1, 8:1, and 10:1. A reaction temperature of step (2) may be 40°C to 150°C, for example, 50°C, 80°C, 100°C, and 120°C. A pressure of hydrogen in step (2) may be 0.5 MPa to 5 MPa, for example, 1 MPa, 1.5 MPa, 2 MPa, and 2.5 MPa.

In step (2), a compound of formula E, serving as a raw material of a reaction, may be used as the solvent. After the completion of the reaction in step (2), a reaction solution is subjected to filtration and vacuum distillation to remove light components, thereby obtaining a compound of formula A.

In the present invention, liquid chromatography (LC) or gas chromatography (GC) may be used to determine whether each step of the reaction has reached the endpoint, so as to confirm the appropriate reaction time.

The antidegradant composition of the present invention comprises two or more compounds of formula A, or consists of two or more compounds of formula A.

In some embodiments, the antidegradant composition of the present invention comprises two or more compounds selected from a compound of formula I, a compound of formula II, a compound of formula IV, a compound of formula V, a compound of formula VII, and a compound of formula VIII:

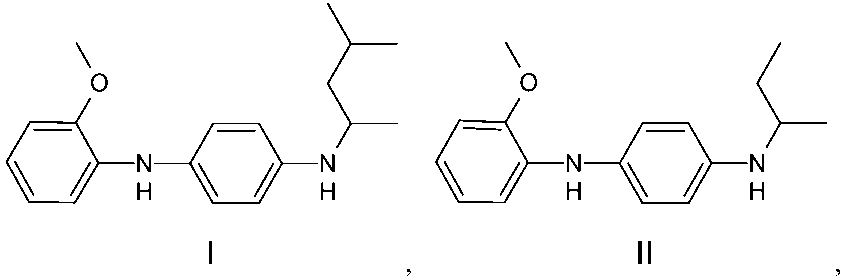

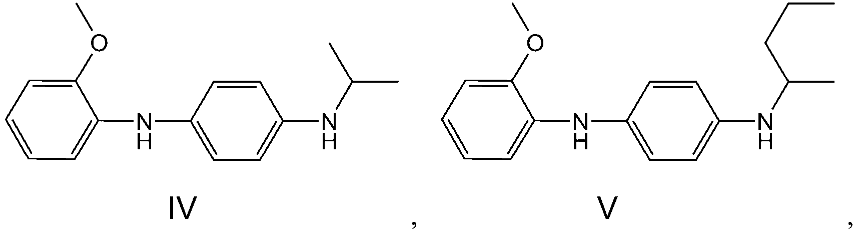

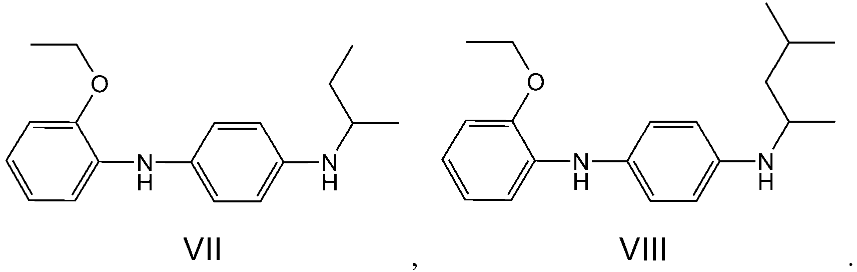

In some embodiments, the antidegradant composition of the present invention comprises two compounds of formula A or consists of two compounds of formula A; a mass ratio of the two compounds of formula A may be 1:4 to 4:1, for example, 1:3, 1:2, 1:1.5, 1:1, 1.5:1, 2:1, and 3:1.

In some preferred embodiments, the antidegradant composition comprises a compound of formula I and a compound of formula II, or consists of a compound of formula I and a compound of formula II. In the antidegradant composition of the present invention, a mass ratio of a compound of formula I to a compound of formula II is preferably 1:4 to 4:1, for example, 1:3, 1:2, 1:1.5, 1:1, 1.5:1, 2:1, and 3:1. Controlling the mass ratio of a compound of formula I to a compound of formula II within the aforementioned range is conducive to exerting the synergistic effect of the two in improving the ozone aging resistance of the rubber composition.

In some other preferred embodiments, the antidegradant composition comprises a compound of formula IV and a compound of formula V, or consists of a compound of formula IV and a compound of formula V. In the antidegradant composition of the present invention, a mass ratio of a compound of formula IV to a compound of formula V is preferably 1:4 to 4:1, for example, 1:3, 1:2, 1:1.5, 1:1, 1.5:1, 2:1, and 3:1. Controlling the mass ratio of a compound of formula IV to a compound of formula V within the aforementioned range is conducive to exerting the synergistic effect of the two in improving the ozone aging resistance of the rubber composition.

In some other preferred embodiments, the antidegradant composition comprises a compound of formula VII and a compound of formula VIII, or consists of a compound of formula VII and a compound of formula VIII. In the antidegradant composition of the present invention, a mass ratio of a compound of formula VII to a compound of formula VIII is preferably 1:4 to 4:1, for example, 1:3, 1:2, 1:1.5, 1:1, 1.5:1, 2:1, and 3:1. Controlling the mass ratio of a compound of formula VII to a compound of formula VIII within the aforementioned range is conducive to exerting the synergistic effect of the two in improving the ozone aging resistance of the rubber composition.

The raw materials of a rubber composition usually comprise a diene elastomer, a reinforcing filler, an antidegradant, and a crosslinking agent. Herein, a rubber composition includes unvulcanized rubber and vulcanized rubber. Unvulcanized rubber can be converted into vulcanized rubber through vulcanization (curing).

The raw materials of a rubber composition of the present invention comprise a diene elastomer and an antidegradant composition of the present invention. Based on 100 parts by mass of the diene elastomer, an amount of an antidegradant composition of the present invention may be 1 to 5 parts by mass, preferably 1 to 4 parts by mass, for example, 1.5 parts by mass, 2 parts by mass, 2.5 parts by mass, 3 parts by mass, and 3.5 parts by mass. Controlling the amount of an antidegradant composition of the present invention within the aforementioned range is conducive to ensuring the effect of improving ozone aging resistance at a relatively low dosage. Herein, unless otherwise specified, the amounts of other components in the raw materials of the rubber composition are calculated based on 100 parts by mass of the diene elastomer in the raw materials of the rubber composition.

Herein, a diene elastomer refers to an elastomer whose monomers comprise dienes (e.g., butadiene, isoprene). The diene elastomers suitable for the present invention may be various diene elastomers known in the art, including but not limited to one or more selected from natural rubber (NR), butadiene rubber (BR), polyisoprene rubber, styrene-butadiene rubber (SBR), chloroprene rubber (CR), nitrile-butadiene rubber (NBR), isoprene/butadiene copolymers, isoprene/styrene copolymers, and isoprene/butadiene/styrene copolymers. In some preferred embodiments, the diene elastomer comprises or consists of natural rubber and butadiene rubber. The mass ratio of natural rubber to butadiene rubber is preferably 1:2 to 2:1, for example, 1:1.5 to 1.5:1, 4.5:5.5 to 5.5:4.5, and 1:1.

The antidegradant contained in the rubber composition of the present invention comprises an antidegradant composition of the present invention. In some embodiments, a rubber composition of the present invention does not contain any other antidegradants except the antidegradant composition of the present invention. In the raw materials of a rubber composition of the present invention, an amount of an antidegradant composition of the present invention may be 1 to 5 parts by mass, preferably 1 to 4 parts by mass, for example, 1.5 parts by mass, 2 parts by mass, 2.5 parts by mass, 3 parts by mass, and 3.5 parts by mass, more preferably 2 to 3 parts by mass.

The raw materials of the rubber composition of the present invention may comprise a reinforcing filler. In the raw materials of the rubber composition of the present invention, the amount of the reinforcing filler may be 30 to 70 parts by mass, for example, 40 parts by mass, 45 parts by mass, 50 parts by mass, 55 parts by mass, and 60 parts by mass. The reinforcing fillers suitable for the present invention may be conventional reinforcing fillers used in rubber compositions, including but not limited to one or more selected from carbon black, silica, titanium oxide, calcium carbonate, magnesium carbonate, aluminum hydroxide, magnesium hydroxide, clay, and talc. In some preferred embodiments, the reinforcing filler comprises carbon black, or the reinforcing filler is carbon black.

The raw materials of the rubber composition of the present invention comprise a crosslinking agent, such as sulfur. In the raw materials of the rubber composition of the present invention, the amount of sulfur may be 0.5 to 3 parts by mass, for example, 1 part by mass, 1.5 parts by mass, 2 parts by mass, and 2.5 parts by mass.

The raw materials of the rubber composition of the present invention may further comprise other components commonly used in rubber compositions, including but not limited to one or more selected from softeners, protective waxes, activators, and accelerators.

Softeners can be used to improve the processing performance of the rubber composition. Softeners may comprise petroleum-based softeners, such as naphthenic oil, aromatic oil, processing oil, lubricating oil, paraffin, liquid paraffin, petroleum asphalt, petrolatum, and the like; they may also comprise fatty oil softeners, such as stearic acid, castor oil, linseed oil, rapeseed oil, coconut oil, waxes (e.g., beeswax, carnauba wax, and lanolin), tall oil, linoleic acid, palmitic acid, lauric acid, and the like. In the raw materials of the rubber composition of the present invention, the amount of the softener may be 2 to 15 parts by mass, for example, 3 parts by mass, 5 parts by mass, 7 parts by mass, 8 parts by mass, 9 parts by mass, 10 parts by mass, 11 parts by mass, and 13 parts by mass. In some preferred embodiments, the softener comprises or consists of aromatic oil and stearic acid. The mass ratio of aromatic oil to stearic acid may be 1:1 to 5:1, for example, 2:1, 2.5:1, 3:1, 3.5:1, and 4:1. In some preferred embodiments, in the raw materials of the rubber composition of the present invention, the amount of aromatic oil is 1 to 10 parts by mass, for example, 2 parts by mass, 3 parts by mass, 4 parts by mass, 5 parts by mass, 6 parts by mass, 7 parts by mass, 8 parts by mass, and 9 parts by mass; the amount of stearic acid is 1 to 5 parts by mass, for example, 1.5 parts by mass, 2 parts by mass, 2.5 parts by mass, 3 parts by mass, and 4 parts by mass.

Protective wax can migrate from the interior of the rubber to the surface of the rubber to form a wax film, functioning to isolate the rubber of the surface from the external environment. Protective wax is optionally added. When the raw materials of the rubber composition of the present invention comprise protective wax, the amount of protective wax may be 1 to 5 parts by mass, for example, 1.5 parts by mass, 2 parts by mass, 3 parts by mass, and 4 parts by mass.

Activators can function to accelerate the vulcanization rate, enhance the thermal conductivity, wear resistance, and tear resistance of the rubber. In the raw materials of the rubber composition of the present invention, the amount of the activator may be 1 to 10 parts by mass, for example, 2 parts by mass, 3 parts by mass, 4 parts by mass, 5 parts by mass, 6 parts by mass, 7 parts by mass, 8 parts by mass, and 9 parts by mass. In some preferred embodiments, the activator comprises ZnO, or the activator is ZnO. In some preferred embodiments, in the raw materials of the rubber composition of the present invention, the amount of ZnO is 3 to 8 parts by mass, for example, 4 parts by mass, 5 parts by mass, 6 parts by mass, and 7 parts by mass.

Accelerators usually refer to vulcanization accelerators, and may be selected from one or more of sulfonamide vulcanization accelerators, thiazole vulcanization accelerators, thiuram vulcanization accelerators, thiourea vulcanization accelerators, guanidine vulcanization accelerators, dithiocarbamate vulcanization accelerators, aldehyde-amine vulcanization accelerators, aldehyde-ammonia vulcanization accelerators, imidazoline vulcanization accelerators, and xanthate acid vulcanization accelerators. In the raw materials of the rubber composition of the present invention, the amount of the accelerator may be 0.2 to 2 parts by mass, for example, 0.5 parts by mass, 0.6 parts by mass, 0.8 parts by mass, 1 part by mass, and 1.5 parts by mass. In some preferred embodiments, the accelerator is accelerator NS (N-tert-butylbenzothiazole-2-sulphenamide).

In addition, when necessary, a plasticizer may also be used in the rubber composition, such as DMP (dimethyl phthalate), DEP (diethyl phthalate), DBP (dibutyl phthalate), DHP (diheptyl phthalate), DOP (dioctyl phthalate), DINP (diisononyl phthalate), DIDP (diisodecyl phthalate), BBP (butyl benzyl phthalate), DWP (dilauryl phthalate), and DCHP (dicyclohexyl phthalate), and the like. The dosage of the plasticizer may be a conventional dosage in the art.

In some preferred embodiments, the raw materials of the rubber composition of the present invention comprise: 100 parts by mass of a diene elastomer, 0.5 to 3 parts by mass of sulfur, 1 to 5 parts by mass of the antidegradant composition of the present invention, 30 to 70 parts by mass of carbon black, 1 to 10 parts by mass of ZnO, 1 to 5 parts by mass of stearic acid, 1 to 10 parts by mass of aromatic oil, and 0.2 to 2 parts by mass of an accelerator, or consist of the above components; wherein the diene elastomer preferably comprises natural rubber and butadiene rubber in a mass ratio of 1:2 to 2:1, and the accelerator is preferably accelerator NS.

The unvulcanized rubber of the present invention may be prepared by conventional rubber mixing methods, for example, by a two-stage mixing process: first-stage thermomechanical (e.g., internal mixer) mixing, mixing the raw materials of the rubber composition excluding the crosslinking agent and accelerator, and kneading the entire mixture until a maximum temperature between 110°C and 190°C is reached to obtain the first-stage rubber; second-stage thermomechanical (e.g., open mill) mixing, after the first-stage rubber cooled down to below 100°C, mixing the first-stage rubber with the crosslinking agent and accelerator until a maximum temperature below 110°C is reached to obtain the second-stage rubber, i.e., the unvulcanized rubber.

Unvulcanized rubber can be vulcanized (cured) to obtain vulcanized rubber. The temperature of vulcanization is usually 130°C to 200°C, for example, 140°C to 160°C or 145°C ± 5°C; the time for vulcanization depends on the temperature of vulcanization, vulcanization system, and vulcanization kinetics, and is usually 15 to 60 minutes, for example, 20 to 40 minutes and 30 minutes ± 5 minutes. Before vulcanization, an unvulcanized rubber may be calendered to a certain thickness, for example, about 2mm.

The rubber composition of the present invention is used in rubber products, especially tires. Compared with a separate use of antidegradant 6PPD or only one compound of formula A, a use of the antidegradant composition of the present invention-particularly the combined use of a compound of formula I and a compound of formula II, the combined use of a compound of formula IV and a compound of formula V, and the combined use of a compound of formula VII and a compound of formula VIII-can significantly improve the ozone aging resistance of rubber products. Therefore, the present invention also provides a rubber product containing the rubber composition described herein. The rubber product may be a tire, a rubber overshoe, a sealing strip, an acoustic panel, or a crash pad, etc. The rubber product is preferably a tire, such as a sidewall rubber, a tread rubber, or the like.

The present invention further provides a use of an antidegradant composition of the present invention-such as the combination of a compound of formula I and a compound of formula II, the combination of a compound of formula IV and a compound of formula V, and the combination of a compound of formula VII and a compound of formula VIII-in improving the ozone aging resistance of a rubber composition or a rubber product, as well as a method for improving the ozone aging resistance of a rubber composition or a rubber product. Preferably, the use or method of the present invention comprises: adding 1 to 5 parts by mass of the antidegradant composition of the present invention to the raw materials of the rubber composition, based on 100 parts by mass of the diene elastomer contained in the rubber composition. In the use or method of the present invention, the raw material composition of the rubber composition is preferably as described in any embodiment herein.

The present invention will be illustrated by way of specific examples below. It should be understood that these examples are merely explanatory and is not intended to limit the scope of the present invention. Unless otherwise specified, the methods, reagents, and materials used in the examples are conventional methods, reagents, and materials in the art. The formulations used in the examples are commercially available.

The raw materials used in the examples are from the following sources: natural rubber (SCR5), Xishuangbanna Sinochem Rubber Co., Ltd.; butadiene rubber (BR9000), Shandong Yuhuang Chemical Co., Ltd.; antidegradant 6PPD, Sennics Co., Ltd.; carbon black N550, zinc oxide, stearic acid (SA), aromatic oil, sulfur (S) and accelerator NS are all common raw materials in the rubber industry.

### Preparation Example 1: synthesis of the compound of formula I

### (1) Synthesis of the compound of formula III

400.2g (3.25mol) of 2-methoxyaniline and 200.2g (0.55mol) of a 25% aqueous solution of tetramethylammonium hydroxide (TMAOH) are added into a 1000mL four-necked flask. The mixture is stirred and heated to 40-50°C then dehydrated by vacuum distillation to allow TMAOH to form a salt with 2-methoxyaniline. During this process, the reaction solution gradually turns from yellow to reddish-brown. The temperature is gradually increased to 75°C; when the fraction is approximately 100g, 61.55g (0.50mol) of nitrobenzene is added dropwise during distillation under reduced pressure (-0.097MPa) at 75°C, with the dropping time lasting about 3 hours. After addition is complete, the solution is kept at this temperature for 1h. The reaction is monitored by LC until the nitrobenzene is completely reacted, yielding a condensate.

The above condensate is transferred to a 1000mL stainless steel reactor, 100g of deionized water and 60g of skeletal nickel catalyst are added. The reactor is purged with hydrogen three times, heated to 65°C, and pressurized to 1.5MPa for reaction. The reaction is monitored by LC until the nitro and nitroso compounds are completely reduced. The reaction solution is filtered, washed with water, and separated into phases; the aqueous phase is recycled after concentration, and the organic phase is subjected to vacuum distillation to obtain 84.6g of compound III (yield is approximately 79.5%) with a GC-detected content >99.2%. 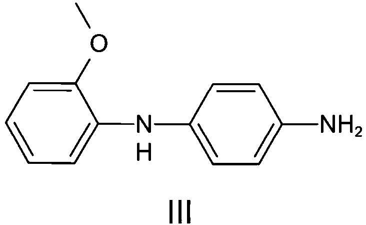
Molecular formula: C₁₃H₁₄N₂O
LC-MS(m/z): 214.26(M-H⁺).

### (2) Synthesis of the compound of formula I

50g (0.23mol) of compound III, 100g (1.0mol) of 4-methyl-2-pentanone, and 1.0g of Pt/C catalyst are added into a 500mL high-pressure reactor. The reactor is heated to 90°C, purged with hydrogen, and then pressurized to 1.2MPa for reaction. The reaction is stopped and cooled down when the content of compound III is <0.1% as detected by GC. The mixture is filtered and subjected to vacuum distillation to remove light components such as water and 4-methyl-2-pentanone, yielding 65.1g of compound I (yield is approximately 95%) with a GC-detected content >98.2%. Appearance: Purplish-brown liquid. 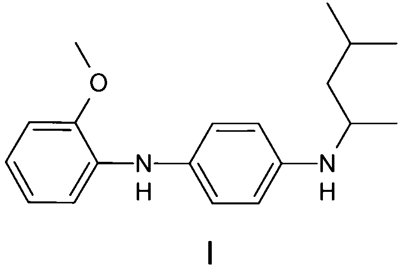
Molecular formula: C₁₉H₂₆N₂O
LC-MS(m/z): 298.42(M-H⁺).

### Preparation Example 2: synthesis of the compound of formula II

### (1) Synthesis of the compound of formula III

The synthesis of compound III is the same as step (1) in Preparation Example 1.

### (2) Synthesis of the compound of formula II

81.5g (0.38mol) of compound III, 117g (1.62mol) of 2-butanone, and 1.0g of Pt/C catalyst are added into a 500mL high-pressure reactor. The reactor is heated to 70°C, purged with hydrogen, and then pressurized to 1.2MPa for reaction. The reaction is stopped and cooled down when the content of compound III is <0.1% as detected by GC. The mixture is filtered and subjected to vacuum distillation to remove light components such as water and 2-butanone, yielding 99.5g of compound II (yield is approximately 97%) with a GC-detected content >97.2%. Appearance: Purple liquid. 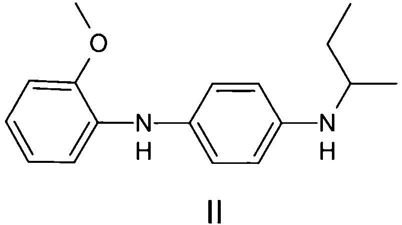
Molecular formula: C₁₇H₂₂N₂O
LC-MS(m/z): 270.37 (M-H⁺).

### Preparation Example 3: synthesis of the compound of formula IV

### (1) Synthesis of the compound of formula III

The synthesis of compound III is the same as step (1) in Preparation Example 1.

### (2) Synthesis of the compound of formula IV

92g (0.43mol) of compound III, 106g (1.83mol) of acetone, and 1.0g of Pt/C catalyst are added into a 500mL high-pressure reactor. The reactor is heated to 75°C, purged with hydrogen, and then pressurized to 1.2MPa for reaction. The reaction is stopped and cooled down when the content of compound III is <0.1% as detected by GC. The mixture is filtered and subjected to vacuum distillation to remove light components such as water and acetone, yielding 106.1g of compound IV (yield is approximately 96%) with a GC-detected content >97.6%. Appearance: Purple liquid. 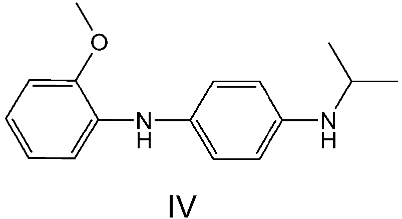
Molecular formula: C₁₆H₂₀N₂O
LC-MS(m/z): 256.35(M-H⁺).

### Preparation Example 4: synthesis of the compound of formula V

### (1) Synthesis of the compound of formula III

The synthesis of compound III is the same as step (1) in Preparation Example 1.

### (2) Synthesis of the compound of formula V

62g (0.29mol) of compound III, 107g (1.24mol) of 2-pentanone, and 1.0g of Pt/C catalyst are added into a 500mL high-pressure reactor. The reactor is heated to 80°C, purged with hydrogen, and then pressurized to 1.2MPa for reaction. The reaction is stopped and cooled down when the content of compound III is <0.1% as detected by GC. The mixture is filtered and subjected to vacuum distillation to remove light components such as water and 2-pentanone, yielding 79.2g of compound V (yield is approximately 96%) with a GC-detected content >98.2%. Appearance: Purple liquid. 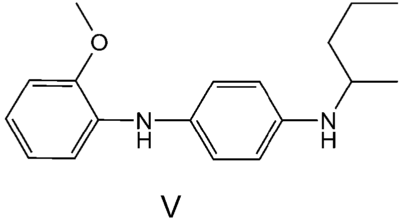
Molecular formula: C₁₈H₂₄N₂O
LC-MS(m/z): 284.40(M-H⁺).

### Preparation Example 5: synthesis of the compound of formula VII

### (1) Synthesis of the compound of formula VI

220g (1.6mol) of 2-ethoxyaniline and 80g (0.22mol) of a 25% aqueous solution of tetramethylammonium hydroxide (TMAOH) are added into a 500mL four-necked flask. The mixture is stirred and heated to 50°C, then dehydrated by vacuum distillation to allow TMAOH to form a salt with 2-ethoxyaniline. During this process, the reaction solution gradually turns from yellow to reddish-brown. The temperature is gradually increased to 75°C; when the fraction is approximately 100g, 24.6g (0.2mol) of nitrobenzene is added dropwise during distillation under reduced pressure (-0.097MPa) at 78°C, with the dropping time lasting about 3 hours. After addition is complete, the solution is kept at this temperature for 1h. The reaction is monitored by LC until the nitrobenzene is completely reacted, yielding a condensate.

The above condensate is transferred to a 500mL stainless steel reactor, 30g of deionized water and 20g of skeletal nickel catalyst are added. The reactor is purged with hydrogen three times, heated to 69°C, and pressurized to 1.6MPa for reaction. The reaction is monitored by LC until the nitro and nitroso compounds are completely reduced. The reaction solution is filtered, washed with water, and separated into phases; the aqueous phase is recycled after concentration, and the organic phase is subjected to vacuum distillation to obtain 34.1g of intermediate compound VI (yield is approximately 75%) with a GC-detected content >98.5%. 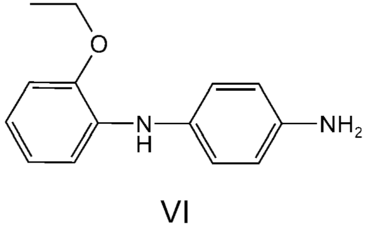
Molecular formula: C₁₄H₁₆N₂O
LC-MS(m/z): 228.26(M-H⁺).

### (2) Synthesis of the compound of formula VII

30g (0.13mol) of compound VI, 43.2g (0.6mol) of 2-butanone, and 0.6g of Pt/C catalyst are added into a 500mL high-pressure reactor. The reactor is heated to 70°C, purged with hydrogen, and then pressurized to 1.2MPa for reaction. The reaction is stopped and cooled down when the content of compound VI is <0.1% as detected by GC. The mixture is filtered and subjected to vacuum distillation to remove light components such as water and 2-butanone, yielding 35.4g of compound VII (yield is approximately 96%) with a GC-detected content >97.5%. Appearance: Reddish-brown liquid. 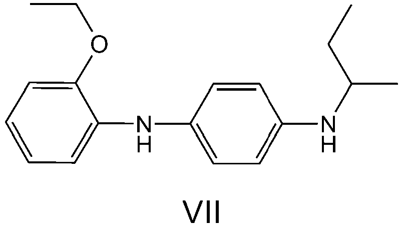
Molecular formula: C₁₈H₂₄N₂O
LC-MS(m/z): 284.40 (M-H⁺).

### Preparation Example 6: synthesis of the compound of formula VIII

### (1) Synthesis of the compound of formula VI

The synthesis of compound VI is the same as step (1) in Preparation Example 5.

### (2) Synthesis of the compound of formula VIII

30g (0.13mol) of compound VI, 78g (0.8mol) of 4-methyl-2-pentanone, and 0.8g of Pt/C catalyst are added into a 500mL high-pressure reactor. The reactor is heated to 90°C, purged with hydrogen, and then pressurized to 1.8MPa for reaction. The reaction is stopped and cooled down when the content of compound VI is <0.1% as detected by GC. The mixture is filtered and subjected to vacuum distillation to remove light components such as water and 4-methyl-2-pentanone, yielding 39.8g of compound VIII (yield is approximately 98%) with a GC-detected content >98.0%. Appearance: Reddish-brown liquid. 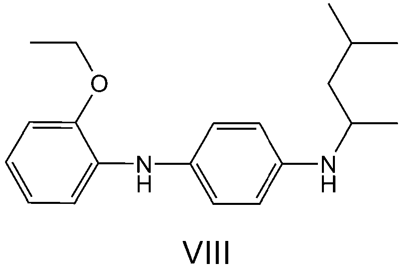
Molecular formula: C₂₀H₂₈N₂O
LC-MS(m/z): 312.46(M-H⁺).

### Example 1

The rubber compositions of 1-14 are prepared using the following process according to the formulations shown in Table 1 and Table 2:
(1) Natural rubber and butadiene rubber are added to an internal mixer; subsequently, carbon black, zinc oxide, stearic acid, aromatic oil and antidegradants (antidegradant IPPD, a compound of formula II, a compound of formula I, a compound of formula IV, a compound of formula V, a compound of formula VII, and/or a compound of formula VIII) are added. The mixture is kneaded until the temperature of the rubber composition reaches 130°C and then discharged to obtain a first-stage rubber.
(2) After the first-stage rubber is cooled down to 50°C, it is milled on an open mill, accelerator NS and sulfur are added, and the mixture is kneaded until the temperature of the rubber composition reaches 70°C and then sheeted out to obtain the second-stage rubber.
(3) The second-stage rubber is calendered into sheets with a thickness of 2 mm, and then vulcanized (145°C×30min) to obtain vulcanized rubber.

**Table 1: Formulation of Rubber Composition (unit: parts by mass)**

| Number of rubber composition | 1 | 2 | 3 | 4 | 5 | 6 | 7 | 8 |
|---|---|---|---|---|---|---|---|---|
| SCR5 | 50.0 | 50.0 | 50.0 | 50.0 | 50.0 | 50.0 | 50.0 | 50.0 |
| BR9000 | 50.0 | 50.0 | 50.0 | 50.0 | 50.0 | 50.0 | 50.0 | 50.0 |
| Carbon Black N550 | 50.0 | 50.0 | 50.0 | 50.0 | 50.0 | 50.0 | 50.0 | 50.0 |
| ZnO | 5.0 | 5.0 | 5.0 | 5.0 | 5.0 | 5.0 | 5.0 | 5.0 |
| SA | 2.0 | 2.0 | 2.0 | 2.0 | 2.0 | 2.0 | 2.0 | 2.0 |
| Aromatic oil | 5.0 | 5.0 | 5.0 | 5.0 | 5.0 | 5.0 | 5.0 | 5.0 |
| Antidegradant 6PPD | 2.5 | 0 | 0 | 0 | 0 | 0 | 0 | 0 |
| A compound of formula II | 0 | 2.5 | 0 | 0.5 | 1.0 | 1.25 | 1.5 | 2.0 |
| A compound of formula I | 0 | 0 | 2.5 | 2.0 | 1.5 | 1.25 | 1.0 | 0.5 |
| Accelerator NS | 0.8 | 0.8 | 0.8 | 0.8 | 0.8 | 0.8 | 0.8 | 0.8 |
| S | 1.5 | 1.5 | 1.5 | 1.5 | 1.5 | 1.5 | 1.5 | 1.5 |
| Total | 166.8 | 166.8 | 166.8 | 166.8 | 166.8 | 166.8 | 166.8 | 166.8 |

**Table 2: Formulation of Rubber Composition (unit: parts by mass)**

| Number of rubber composition | 9 | 10 | 11 | 12 | 13 | 14 |
|---|---|---|---|---|---|---|
| SCR5 | 50.0 | 50.0 | 50.0 | 50.0 | 50.0 | 50.0 |
| BR9000 | 50.0 | 50.0 | 50.0 | 50.0 | 50.0 | 50.0 |
| Carbon Black N550 | 50.0 | 50.0 | 50.0 | 50.0 | 50.0 | 50.0 |
| ZnO | 5.0 | 5.0 | 5.0 | 5.0 | 5.0 | 5.0 |
| SA | 2.0 | 2.0 | 2.0 | 2.0 | 2.0 | 2.0 |
| Aromatic oil | 5.0 | 5.0 | 5.0 | 5.0 | 5.0 | 5.0 |
| A compound of formula IV | 1.25 | 0 | 2.5 | 0 | 0 | 0 |
| A compound of formula V | 1.25 | 0 | 0 | 2.5 | 0 | 0 |
| A compound of formula VII | 0 | 1.25 | 0 | 0 | 2.5 | 0 |
| A compound of formula VIII | 0 | 1.25 | 0 | 0 | 0 | 2.5 |
| Accelerator NS | 0.8 | 0.8 | 0.8 | 0.8 | 0.8 | 0.8 |
| S | 1.5 | 1.5 | 1.5 | 1.5 | 1.5 | 1.5 |
| Total | 166.8 | 166.8 | 166.8 | 166.8 | 166.8 | 166.8 |

### Test Example 1: Vulcanization Characteristics Test and Thermal-Oxidative Aging Test

The vulcanization characteristics t10 and t90 are determined in accordance with GB/T 16584-1996 "Rotorless Rheometers for Rubber". The thermal-oxidative aging test of the rubber composition is conducted in accordance with GB/T 3512-2014 "Accelerated Aging and Heat Resistance Test for Vulcanized Rubber or Thermoplastic Rubber by Hot Air" under the test conditions of 100°C×48 hours. The test standards for elongation at break and tensile strength before and after aging are GB/T 528-2009 "Determination of Tensile Stress-Strain Properties of Vulcanized Rubber or Thermoplastic Rubber". The results are shown in Tables 3-4.

**Table 3: Vulcanization characteristics and physical properties before and after Thermal-Oxidative Aging**

| Number of rubber composition | 1 | 2 | 3 | 4 | 5 | 6 | 7 | 8 |
|---|---|---|---|---|---|---|---|---|
| t10 | 8.99 | 8.53 | 8.76 | 8.62 | 8.60 | 8.68 | 8.67 | 8.72 |
| t90 | 16.69 | 16.05 | 16.34 | 16.12 | 16.08 | 16.23 | 16.27 | 16.37 |
| Elongation at break before aging (%) | 534 | 517 | 505 | 510 | 527 | 538 | 517 | 531 |
| Tensile strength before aging (MPa) | 18.9 | 19.2 | 19.1 | 18.9 | 19.0 | 19.3 | 19.7 | 18.5 |
| Elongation at Break after Aging at 100°C×48h (%) | 393 | 351 | 378 | 386 | 412 | 401 | 408 | 410 |
| Tensile strength after Aging at 100°C×48h (MPa) | 16.7 | 15.5 | 15.7 | 14.5 | 15.8 | 16.3 | 16.0 | 15.9 |

**Table 4: Vulcanization characteristics and physical properties before and after Thermal-Oxidative Aging**

| Number of rubber composition | 9 | 10 | 11 | 12 | 13 | 14 |
|---|---|---|---|---|---|---|
| t10 | 9.00 | 8.76 | 8.61 | 8.59 | 8.71 | 8.66 |
| t90 | 16.72 | 16.26 | 16.01 | 16.04 | 16.12 | 16.44 |
| Elongation at break before aging (%) | 528 | 538 | 529 | 526 | 539 | 531 |
| Tensile strength before aging (MPa) | 19.2 | 19.5 | 19.1 | 18.7 | 18.4 | 19.0 |
| Elongation at Break after Aging at 100°C×48h (%) | 377 | 364 | 371 | 365 | 375 | 359 |
| Tensile strength after Aging at 100°C×48h (MPa) | 15.3 | 15.5 | 15.3 | 15.8 | 16.1 | 15.2 |

### Test Example 2: Ozone Aging Test

The ozone aging test of the rubber composition is conducted in an ozone aging test chamber in accordance with ISO 1431-1:2004 " Ozone Cracking Resistance of Vulcanized Rubber or Thermoplastic Rubber". The test conditions are as follows: a volume concentration of ozone is 50×10⁻⁸, a temperature is (40±2)°C, and a humidity is (50±5)%. For the static test, the sample is pre-stretched by 20% and operated at fixed time intervals to observe the cracking of the sample. For the dynamic test, the sample is pre-stretched by 10% with a dynamic tensile strain of 10% and a frequency of 0.5Hz, and operated at fixed time intervals to observe the cracking of the sample. The results of the static ozone aging test are shown in Tables 5-6, and the results of the dynamic ozone aging test are shown in Tables 7-8. The explanation of ozone crack grades in Tables 5-8 is shown in Table 9.

**Table 5: Results of Crack Grade of Static Ozone Aging**

| Cumulative operating time /h | Rubber composition 1 | Rubber composition 2 | Rubber composition 3 | Rubber composition 4 | Rubber composition 5 | Rubber composition 6 | Rubber composition 7 | Rubber composition 8 |
|---|---|---|---|---|---|---|---|---|
| 2 | 1 | 1 | 1 | 0 | 0 | 0 | 0 | 1 |
| 4 | 1 | 1 | 1 | 1 | 1 | 0 | 1 | 1 |
| 8 | 1 | 1 | 1 | 1 | 1 | 1 | 1 | 1 |
| 16 | 1 | 1 | 1 | 1 | 1 | 1 | 1 | 1 |
| 24 | 2 | 2 | 2 | 1 | 1 | 1 | 1 | 1 |
| 48 | 2 | 3 | 2 | 2 | 1 | 1 | 2 | 2 |
| 72 | 3 | 3 | 2 | 2 | 2 | 2 | 2 | 2 |
| 96 | 3 | 4 | 3 | 3 | 2 | 2 | 2 | 3 |
| 120 | 4 | 4 | 4 | 3 | 3 | 2 | 3 | 3 |

**Table 6: Results of Crack Grade of Static Ozone Aging**

| Cumulative operating time /h | Rubber composition 9 | Rubber composition 10 | Rubber composition 11 | Rubber composition 12 | Rubber composition 13 | Rubber composition 14 |
|---|---|---|---|---|---|---|
| 2 | 0 | 0 | 1 | 0 | 1 | 1 |
| 4 | 0 | 1 | 1 | 1 | 1 | 1 |
| 8 | 1 | 1 | 1 | 1 | 1 | 1 |
| 16 | 1 | 1 | 2 | 2 | 1 | 1 |
| 24 | 1 | 1 | 2 | 2 | 1 | 2 |
| 48 | 2 | 2 | 2 | 2 | 2 | 2 |
| 72 | 2 | 2 | 3 | 3 | 2 | 3 |
| 96 | 2 | 2 | 3 | 3 | 2 | 3 |
| 120 | 3 | 3 | 4 | 4 | 3 | 4 |

**Table 7: Results of Crack Grade of Dynamic Ozone Aging**

| Cumulative operating time /h | Rubber compo sition 1 | Rubber compo sition 2 | Rubber compo sition 3 | Rubber compo sition 4 | Rubber compo sition 5 | Rubber compo sition 6 | Rubber compo sition 7 | Rubber compo sition 8 |
|---|---|---|---|---|---|---|---|---|
| 2 | 0 | 0 | 1 | 1 | 1 | 0 | 0 | 0 |
| 4 | 1 | 1 | 1 | 1 | 1 | 1 | 1 | 1 |
| 8 | 1 | 1 | 2 | 1 | 1 | 1 | 1 | 1 |
| 16 | 1 | 2 | 2 | 2 | 1 | 1 | 1 | 2 |
| 24 | 2 | 2 | 2 | 2 | 2 | 1 | 2 | 2 |
| 48 | 2 | 2 | 3 | 2 | 2 | 2 | 2 | 2 |
| 72 | 3 | 2 | 3 | 2 | 2 | 2 | 2 | 2 |
| 96 | 3 | 3 | 4 | 3 | 3 | 2 | 2 | 2 |
| 120 | 4 | 4 | 4 | 3 | 3 | 3 | 3 | 3 |

**Table 8: Results of Crack Grade of Dynamic Ozone Aging**

| Cumulative operating time /h | Rubber composi tion 9 | Rubber composi tion 10 | Rubber composi tion 11 | Rubber composi tion 12 | Rubber composi tion 13 | Rubber composi tion 14 |
|---|---|---|---|---|---|---|
| 2 | 0 | 0 | 1 | 1 | 1 | 1 |
| 4 | 1 | 1 | 1 | 1 | 2 | 2 |
| 8 | 1 | 1 | 2 | 2 | 2 | 2 |
| 16 | 1 | 1 | 2 | 2 | 2 | 2 |
| 24 | 1 | 2 | 2 | 2 | 2 | 2 |
| 48 | 2 | 2 | 2 | 3 | 3 | 3 |
| 72 | 2 | 2 | 3 | 3 | 3 | 3 |
| 96 | 3 | 3 | 4 | 4 | 4 | 4 |
| 120 | 3 | 3 | 4 | 4 | 4 | 4 |

**Table 9: Explanation of Crack Grades in Ozone Aging Test**

| Grade | Explanation |
|---|---|
| 0 | No cracks |
| 1 | Crack width<0.1mm |
| 2 | 0.1mm ≤ Crack width < 0.2mm |
| 3 | 0.2mm ≤ Crack width < 0.4mm |
| 4 | Crack width ≥ 0.4mm |

As can be seen from Tables 3-8, compared with Rubber Compositions 1-3 only containing 6PPD, a compound of formula I, or a compound of formula II, Rubber Compositions 4-8 comprising both a compound of formula I and a compound of formula II exhibit comparable basic physical properties, along with significantly improved static and dynamic ozone aging resistance. Compared with Rubber Compositions 11-12 only containing a compound of formula IV or a compound of formula V, Rubber Composition 9 comprising both a compound of formula IV and a compound of formula V shows comparable basic physical properties and remarkably enhanced static and dynamic ozone aging resistance. Compared with Rubber Compositions 13-14 only containing a compound of formula VII or a compound of formula VIII, Rubber Composition 10 comprising both a compound of formula VII and a compound of formula VIII possesses comparable basic physical properties and improved static and dynamic ozone aging resistance. These results indicate that the combined use of a compound of formula I and a compound of formula II, the combined use of a compound of formula IV and a compound of formula V, as well as the combined use of a compound of formula VII and a compound of formula VIII, have achieved unexpected synergistic effects in improving the static and dynamic ozone aging resistance of rubber compositions.

## Claims

1. An antidegradant composition, wherein the antidegradant composition comprises two or more compounds of formula A: 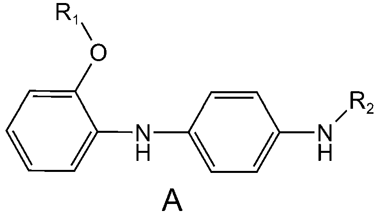 in formula A, R₁ and R₂ are each independently selected from C1-C8 alkyl.

2. The antidegradant composition of claim 1, wherein in formula A, R₁ is selected from C1-C2 alkyl, and R₂ is selected from C3-C6 alkyl.

3. The antidegradant composition of claim 1, wherein the antidegradant composition comprises two or more compounds selected from a compound of formula I, a compound of formula II, a compound of formula IV, a compound of formula V, a compound of formula VII, and a compound of formula VIII:

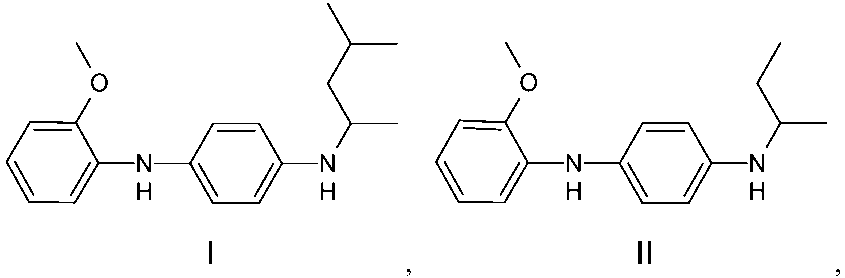

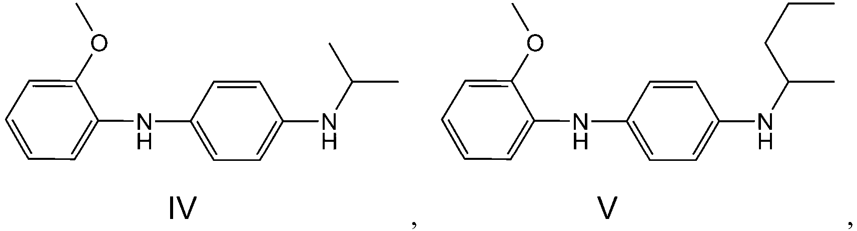

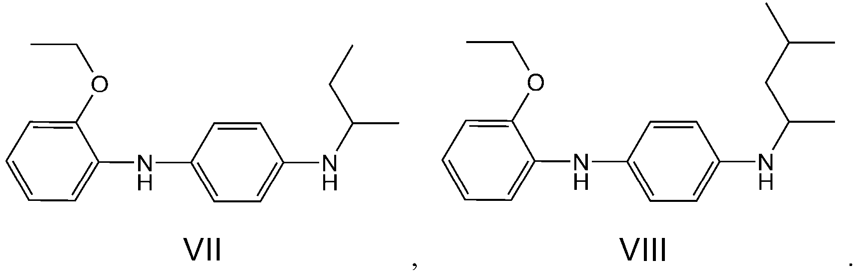

4. The antidegradant composition of claim 3, wherein the antidegradant composition comprises a compound of formula I and a compound of formula II, or comprises a compound of formula IV and a compound of formula V, or comprises a compound of formula VII and a compound of formula VIII;
preferably, in the antidegradant composition, a mass ratio of a compound of formula I to a compound of formula II, a mass ratio of a compound of formula IV to a compound of formula V, or a mass ratio of a compound of formula VII to a compound of formula VIII is 1:4 to 4:1.

5. A rubber composition, wherein raw materials of the rubber composition comprise 100 parts by mass of a diene elastomer and 1 to 5 parts by mass of the antidegradant composition according to any one of claims 1-4.

6. The rubber composition of claim 5, wherein raw materials of the rubber composition comprise 2 to 3 parts by mass of the antidegradant composition.

7. The rubber composition of claim 5, wherein the diene elastomer comprises natural rubber and butadiene rubber; preferably, a mass ratio of the natural rubber to the butadiene rubber is 1:2 to 2:1.

8. The rubber composition of claim 5, wherein the rubber composition has one or more of the following characteristics:
the raw materials of the rubber composition further comprise 30 to 70 parts by mass of a reinforcing filler; preferably, the reinforcing filler is carbon black;
the raw materials of the rubber composition further comprise 1 to 10 parts by mass of an activator; preferably, the activator is zinc oxide;
the raw materials of the rubber composition further comprise 2 to 15 parts by mass of a softener; preferably, the softener comprises aromatic oil and stearic acid in a mass ratio of 1:1 to 5:1;
the raw materials of the rubber composition further comprise 0.5 to 3 parts by mass of sulfur;
the raw materials of the rubber composition further comprise 0.2 to 2 parts by mass of an accelerator, and the accelerator is preferably N-tert-butylbenzothiazole-2-sulphenamide.

9. A rubber product, wherein the rubber product comprises the rubber composition according to any one of claims 5-8; preferably, the rubber product is a tire.

10. Use of the antidegradant composition according to any one of claims 1-4 in improving the ozone aging resistance of a rubber composition or a rubber product.
